# EUROPEAN PATENT SPECIFICATION

(11) **EP 2 042 221 B1**
(45) Date of publication and mention of the grant of the patent: **02.05.2012**
(21) Application number: 07113560.2
(22) Date of filing: 31.07.2007
(51) Int. Cl.: B01D 29/21, A61M 1/36

(54) **Filter for body fluids**
Filter für Körperflüssigkeiten
Filtre pour fluides corporels

(43) Date of publication of application: 01.04.2009
(73) Proprietor: Sorin Group Italia S.r.l., 41037 Mirandola (MO) (IT)
(72) Inventor: Panzani, Ivo, 41037, Mirandola (Modena) (IT); Zaniboni, Andrea, 41030, Quarantoli (Modena) (IT); Mantovani, Marco, 41037, Mirandola (Modena) (IT)
(74) Representative: Freyria Fava, Cristina

(56) References cited:
- EP-A- 0 318 993
- WO-A-00/40319
- WO-A-95/17945
- JP-A- 60 238 111

## Description

### TECHNICAL FIELD

The present invention relates to filters for filtering body fluids, such as blood (or its constituents).

### BACKGROUND

During cardiovascular surgery, a patient's blood is circulated via an extracorporeal circuit. The patient's blood is drained from the body by means of one or two venous cannulas, placed generally in the superior and/or inferior vena cava, and conveyed to an extracorporeal circuit comprising an oxygenator device and an arterial filter. The oxygenated blood is returned to the patient's body by means of an arterial cannula, generally placed in the aorta. The oxygenator may include a heat exchanger to control the blood temperature. An arterial filter is generally located downstream from the oxygenator and is intended to remove any solid or gaseous emboli (e.g., particles or bubbles) from the blood.

Filters known in the art have relatively large overall dimensions and priming volume. These factors, in combination with the priming volume in an oxygenator circuit, may produce a relatively high hemodilution of the patient. The term "hemodilution" defines a situation where the fluid content of blood is increased over the standard levels, which results in a lowered concentration of the blood corpuscular components (e.g., red blood cells). Hemodilution may cause damage to the patient by, for example, resulting in an insufficient amount of oxygen available to the patient's body, which may lead to ischemic processes in various organs.

### SUMMARY

The present invention, *concerns* an arterial filter, which includes a housing provided with blood inlet and outlet ports, a filtering element located within the housing, and a gas vent in the top wall of the housing. The filtering element is generally comprised of a pleated layer or array of layers of a laminar or sheet-like material wrapped in a cylindrical or toroidal configuration. *An arterial filter if this type is known i.a. from EP-A-318 993. The arterial filter according to the instant invention is characterized by having a filtering element wherein* the pleated material includes an alternation of pleats having different lengths. In some embodiments, the filtering material is a synthetic polymer composition mesh or a stainless steel wire mesh.

In an embodiment, the present invention concerns a filter comprising a filtering element made of a pleated layer wrapped in a toroidal configuration, wherein the pleated layer is provided with pleats of different lengths. In a filtering element with pleats of different lengths, the priming volume of the filter can be reduced, while maintaining a constant filtering surface. In an embodiment, the filter according to the present invention facilitates debubbling during set-up, while affording good "microair" handling capabilities.

The present invention, according to yet another embodiment, includes a filter for filtering blood comprising a pleated filtering element. The filtering element includes alternating short pleats and long pleats configured such that a priming volume of the filter decreases significantly with respect to a configuration having pleats of a generally equal length.

### BRIEF DESCRIPTION OF THE DRAWINGS

Figures 1 and 2 show a perspective view and a cross-sectional view of a preferred embodiment of a filter as described herein.

Figures 3 and 4 are a perspective and a top planar view of a filtering element included in a filter as described herein.

Figures 5 and 6 are a perspective and a top planar view of an alternative filtering element included in a filter as described herein.

Figure 7 is a top planar view of a further alternative filtering element included in a filter as described herein.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

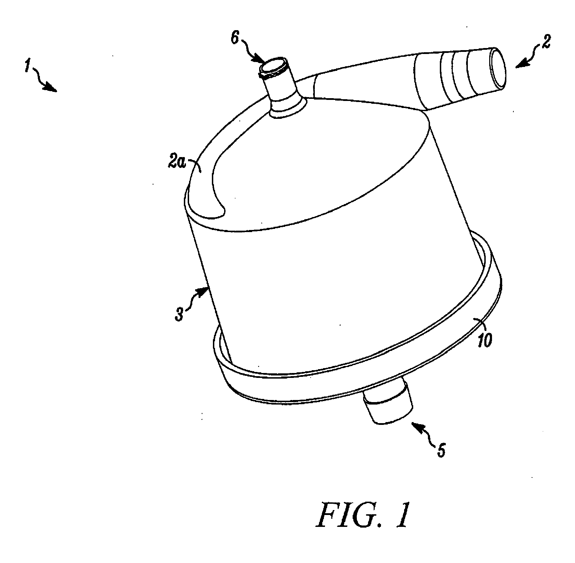

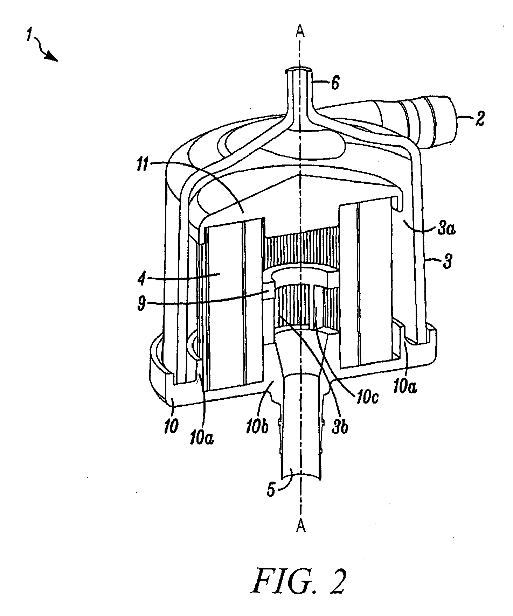

Figures 1 and 2 show view of a filter 1 for filtering body fluids. One such exemplary filter, which will be referred to throughout this description, is an arterial filter. The filter 1 comprises an inverted-cup shaped housing 3 containing a filtering element 4. The housing 3 is closed at its lower end by a bottom element 10. An inlet port 2 and an outlet port 5 enable connection of the filter 1 to a blood circulation circuit. In various exemplary embodiments, such a circuit includes an oxygenator device (not shown). The filter is generally located downstream from the oxygenator and is intended to remove any solid or gaseous emboli (e.g., particles, bubbles, etc.) from the body fluid (e.g., blood). This filtering action is achieved as the fluid flowing in the fluid line from the inlet port 2 to the outlet port 5 traverses the filtering element 4. Any gases removed from the fluid are vented from the housing 3 via a vent port 6, which in various embodiments is arranged at the top of the housing 3.

In the exemplary embodiment shown, the housing 3 has a slightly frusto-conical shape, which tapers along its central longitudinal axis A-A towards a funnel-shaped upper portion. Notably, in other embodiments of the invention, the housing 3 may have a variety of alternative shapes. As shown in Figure 2, the vent port 6 is located at a center of the upper portion. The outer housing 3 may be made of any desired synthetic resin approved for use in contact with body fluids in medical applications (e.g., polycarbonate, polyester, ABS, etc.) and is preferably transparent to allow observation of the inside of the filter 1.

As further shown in Figure 2, the filtering element 4 is centered along the central longitudinal axis A-A and is comprised of a sheet of filtering material which is pleated and then wrapped in a toroidal configuration. According to various embodiments, the filtering material may be a screen mesh filtering element of a type generally known in the art. Likewise, in other embodiments of the invention, the filtering element 4, may have other configurations (e.g., cylindrical). The toroid-shaped filtering element 4 divides the space within the housing 3 in two regions: an external region 3a, corresponding to the space between the external surface of the filtering element 4 and the inner side wall of the housing 3, and an internal region 3b, essentially corresponding to the axial cavity of the toroid-shaped filtering element 4.

As shown, the filtering element 4 is closed at its upper end by a cap 11, which is disposed at a distance from the upper wall of the housing 3 thus defining a fluid inlet chamber into which fluid can flow via the inlet port 2. The axial cavity of the toroid-shaped filtering element 4 in turn defines a fluid outlet chamber from which fluid can flow via the outlet port 5. The entering fluid can thus move from the region 3a to the region 3b by passing through the filtering element 4.

The filtering element 4 is secured within the housing 3 by annular rims 10a and 10b protruding from the bottom element 10 of the housing 3. The filtering element 4 may be further supported by a rim 9 which is connected to the rim 10b through bridges 10c. In various embodiments, the bottom element 10 includes an external annular groove adapted to receive the bottom end of the housing 3, so as to close the housing in a liquid-tight manner.

As shown in Figures 1 and 2, the inlet port 2 is located on the outer circumferential surface of the upper portion of the housing 3 and is in fluid communication with the space between the external surface of the filtering element 4 and the inner side wall of the housing 3 (i.e., the external region 3a). As better illustrated in Figure 1, the housing 3 may be provided with a groove 2a to direct the blood flow incoming from the blood inlet 2 in a non-perpendicular manner against its inner side wall.

The outlet port 5, as shown in Figures 1 and 2, is located in the bottom element 10 concentrically with respect to the central longitudinal axis A-A of the housing 3, in fluid communication with the internal region 3b of the housing 3. In other embodiments of the present invention, the inlet port 2 and outlet port 5 may be disposed in other locations on the housing 3, so long as they are in fluid communication with opposing surfaces of the filtering element 4.

During use, the blood (or other bodily fluid) enters the filter 1 by means of the inlet port 2, which generates a centripetal (i.e., swirling) flow of the blood inside the housing 3. Because of the presence of the cap 11, which is preferably cone-shaped, the incoming blood from the inlet port 2 is diverted radially towards the external surface of the filtering element 4. When traversing the filtering element 4, the blood flows essentially in a perpendicular manner with respect to the external surface of the filtering element 4. The fluid filtered through the filtering element 4 flows into the internal region 3b and out through the outlet port 5.

Such a flow of blood within the filter 1 allows a good separation of the gaseous emboli possibly present therein, facilitating their removal through the gas vent 6, while also preventing any adverse effect on the blood corpuscular components. The "perpendicular" flow of blood through the filtering element 4, which avoids any excessive pressure being applied on the corpuscular elements of blood, plays a significant role in achieving that result.

The filtering element 4 may be made of materials that are acceptable for contact with blood and provide adequate filtration. According to one exemplary embodiment, the filtering element 4 is made of a laminar material with a layered structure including two or three layers of polymeric woven material. In the case of a three-layer structure, the innermost and the outermost layers provide support to the central filtering layer. The filtering capacity is determined by the pore size of the woven materials. According to exemplary embodiments, the supporting layers are generally provided with a pore size in the range of between about 200 and about 1500 micron, and the filtering layer is made of a polyester woven material having a pore size in the range between about 15 and about 40 micron. According to other embodiments of the invention, the pore sizes may be of any size or range of sizes effective for filtering the bodily fluid.

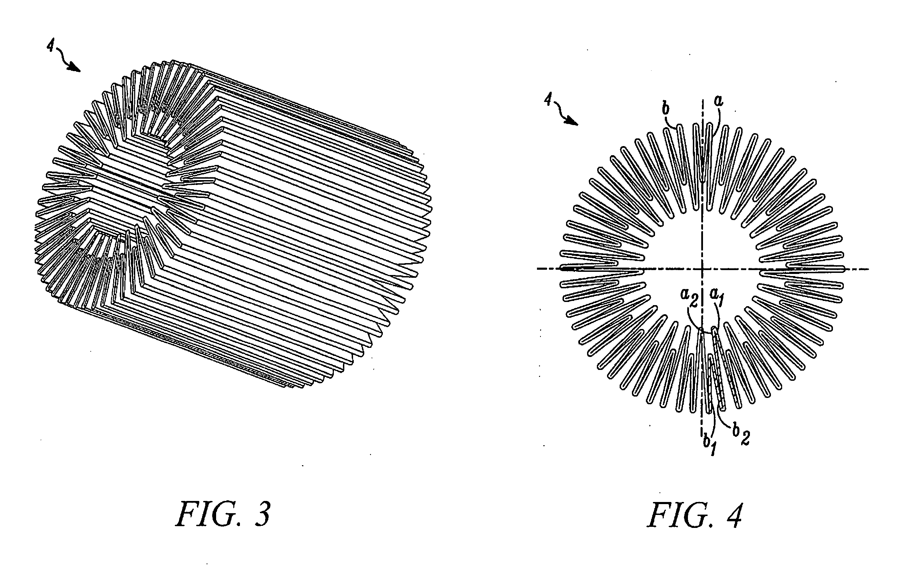

As shown in Figures 3-7, the filtering element 4 is pleated to include pleats of different lengths, namely long and short pleats having length Iₐ and I_{b}, respectively, with Iₐ greater than I_{b}. In various exemplary embodiments, the values for Iₐ and I_{b} are about 15 mm and about 10 mm, respectively.

In the exemplary arrangements shown in the drawings, the term "pleat" designates a generally V-shaped structure comprised of two arms (a₁ and a₂ or, respectively, b₁ and b₂) having their distal ends at the outer cylindrical surface of the filtering element 4 and their proximal ends connected to form the loop portion of the V-shape directed towards the inner cavity of the filtering element 4, so that the proximal inner ends of the "long" pleats and the "short" pleats lie on two different notional inner cylindrical surfaces of the filtering element 4. In other words, the long pleats, having a length Iₐ, define a first, smaller inside diameter of the filtering element 4, and the short pleats, having a length Iₐ, define a second, larger insider diameter of the filtering element 4.

In the exemplary arrangements shown in the drawings, the pleats are symmetrical, in that a₁ = a₂ = Iₐ and b₁ = b₂ = I_{b}. According to alterative embodiments, either or both of the long and the short pleats are asymmetrical (i.e. a₁ ≠ a₂ and/or b₁ ≠ b₂).

According to the embodiment shown in Figures 3 and 4, the sequence (or pattern) of pleats includes one long pleat "a" alternating with one short pleat "b" (i.e., a sequence of the type a,b,a,b,a,b,...). In other words, in the arrangement shown in Figures 3 and 4, the alternation of pleats includes each long pleat interposed between two short pleats (i.e., one short pleat on either side of each long pleat).

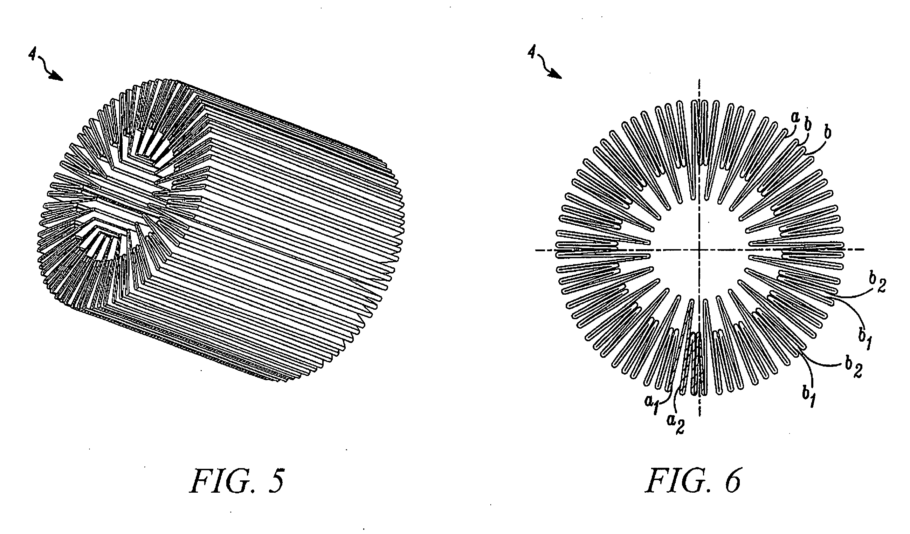

Figures 5 and 6 show an alternative embodiment of the invention wherein the sequence of pleats includes one long pleat "a" alternating with two short pleats "b" (i.e., a sequence of the type a,b,b,a,b,b,...) Thus, in the arrangement of Figures 5 and 6, the alternation of pleats includes each long pleat interposed between two pairs of short pleats. Many other sequence patterns (e.g., a,a,b,a,a,b...) may be adopted in the alternation of pleats depending on specific requirements.

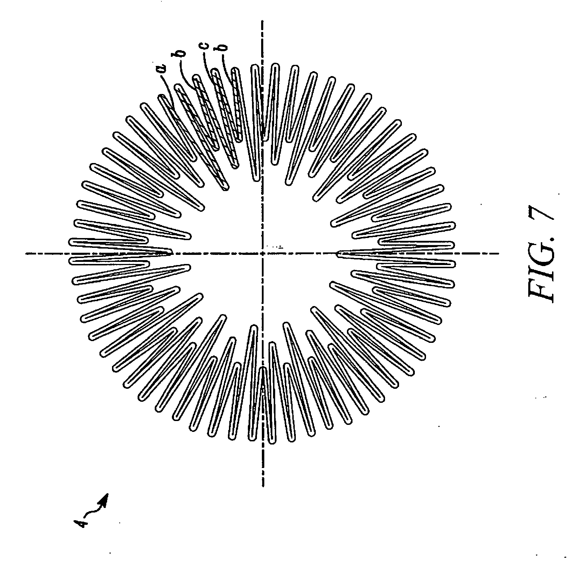

Figure 7 shows yet another alternative embodiment of the filter element 4, according to the present invention. As shown in Figure 7, the filter further includes intermediate length pleats "c" disposed between the long and the short pleats, namely pleats having a length I_{c} which is greater than I_{b} and smaller than Iₐ. According to various exemplary embodiments, the value for I_{c} is 12.5 mm.

Also in this case, the various pleats a, b, c having different lengths Iₐ, I_{b}, I_{c} have distal ends lying on an outer cylindrical surface of the filtering element as well as proximal ends lying on (three) respective distinct notional inner cylindrical surfaces of the filtering element. Similar to the pleats a and b, the pleats c may be either symmetrical or asymmetrical.

In one exemplary configuration of a filtering element 4, which includes intermediate length pleats, each intermediate pleat "c" is interposed between two short pleats "b" and these three pleats (i.e., b,c,b) are interposed between two long pleats "a", so to give rise to a sequence of a,b,c,b,a,b,c,b,a,.... According to various other embodiments, the filtering element 4 includes other sequence patterns (e.g. a,c,b,c,a,c,b,c,a,...) depending on specific requirements.

Table 1 below includes some comparative data relating to two embodiments of the filtering element 4 shown in the drawings (second, third and fourth lines) are given with reference to a conventional filtering element including pleats of equal lengths (first line). Specifically the reference filtering element 4 includes pleats all having a length Iₐ equal to the length of the "long" pleats in the filtering elements according to the arrangement described herein.

**Table 1**

| **Long pleats number** | **lₐ⁽¹⁾ (mm)** | **Short pleats number** | **l_{b}⁽²⁾ (mm)** | **Interm. length pleats number** | **1_{c}⁽³⁾ (mm)** | **dᵢ⁽⁴⁾ (mm)** | **dₑ⁽⁵⁾ (mm)** | **Filtering layer height (mm)** | **Filter surface (cm²)** | **Priming volume (cm³)** | **S/V (cm^{- 1})** |
|---|---|---|---|---|---|---|---|---|---|---|---|
| 48 | 15 | 0 | 0 | 0 | 0 | 25 | 55 | 40 | 576 | 95 | 6.06 |
| 28 | 15 | 28 | 10 | 0 | 0 | 20 | 50 | 40 | 560 | 78.5 | 7.13 |
| 20 | 15 | 40 | 10 | 0 | 0 | 15 | 45 | 40 | 560 | 63.6 | 8.80 |
| 15 | 15 | 30 | 10 | 15 | 12.5 | 18 | 48 | 40 | 570 | 72.4 | 7.87 |

| | | | | | | | | | | | |
|---|---|---|---|---|---|---|---|---|---|---|---|
| (1) Length of the long pleats (2) Length of the short pleats (3) Length of the intermediate length pleats (4) Filtering element internal (innermost) diameter (5) Filtering element external diameter | | | | | | | | | | | |

The data provided in Table 1 demonstrates that, by implementing a filtering element including an array of one long pleat "a" and at least one short pleat "b," it is possible to appreciably reduce the outer diameter of the filtering element (e.g., from 55 to 50 or even 45 mm) without appreciably reducing the filter surface (e.g., from 576 to 560 cm²).

By increasing the number of short pleats from 0 (as in the prior art) up to 40 (as in the embodiment shown in Figures 5 and 6, wherein the pleats array is of the type abb) and consequently decreasing the number of long pleats from 40 to 20, the filter surface remains substantially the same, while the priming volume decreases dramatically, thus decreasing the hemodilution of the patient.

Moreover, by including intermediate length pleats "c" interposed between two short pleats "b," and having these three pleats b,c,b interposed between two long pleats "a" (as in the embodiment shown in Figure 7, wherein the array of pleats is of the type abcb), the filter surface remains substantially the same, while the priming volume decreases again dramatically with respect to the prior art filter, thus decreasing the hemodilution of the patient.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A blood filter (1) comprising a housing (3) having an inlet port (2), an outlet port (5), a vent port (6), and a filtering element (4) disposed therein, wherein the filtering element (4) is made of a porous, pleated material which is acceptable for contact with and provides filtration of blood and is arranged in a cylindrical configuration, such that it defines a sequence of pleats (a, b, c) including first pleats (a) having a first length (Iₐ) and second pleats (b) having a second length (I_{b}), wherein the inlet port (2) and the outlet port (5) are in fluid communication with opposing surfaces of the filtering element (4) and wherein the inlet port (2) is tangentially disposed on the outer circumferential surface of the housing (3), **characterized in that** the first pleats (a) and the second pleats (b) have a different length, wherein the first length (Iₐ) is greater than the second length (I_{b}).

2. The filter (1) according to claim 1 further including third pleats (c), the third pleats (c) having an intermediate length (I_{c}) between the first length (Iₐ) and the second length (I_{b}).

3. The filter (1) according to claim 1, wherein the sequence of pleats includes an alternation of each first pleat (a) interposed between two second pleats (b).

4. The filter (1) according to claim 1, wherein the sequence includes an alternation of each first pleat (a) interposed between two pair of second pleats (b).

5. The filter (1) according to claim 2, wherein the first length (Iₐ) is greater than the second length (I_{b}) and the intermediate length (I_{c}) is intermediate to the first length (Iₐ) and the second length (I_{b}), and further wherein the sequence includes an alternation of each third pleat (c) interposed between two second pleats (b).

6. The filter (1) according to claim 5, wherein the alternation further includes two first pleats (a) disposed adjacent the two second pleats (b).

7. The filter (1) according to claim 1, wherein each of the pleats (a,b,c) have distal ends generally defining a common, notional outer cylindrical surface of the filtering element (4).

8. The filter (1) according to claim 1, wherein the first pleats (a) have proximal ends generally defining a first, notional inner cylindrical surface and the second pleats (b) have proximal ends generally defining a second, notional inner cylindrical surface of the filtering element (4).

9. The filter (1) according to claim 1, wherein the pleats (a, b, c) are symmetrical including two arms of generally equal lengths.

10. The filter (1) according to claim 1, wherein the material has a layered structure including at least one filtering layer.

11. The filter (1) according to claim 10, wherein the material has a three-layer structure including a central filtering layer interposed between two outer support layers.

12. The filter (1) according to claim 1, wherein the first length (Iₐ) is about 15 mm and the second length (I_{b}) is about 10 mm.

13. The filter (1) according to claim 1, wherein the outlet port (5) is disposed on a bottom element (10) and is concentrically disposed with respect to the central longitudinal axis.

14. The filter (1) according to claim 1, further including a cap (11) located on an upper portion of the filter element (4).

15. The filter according to Claim 10, wherein said filtering layer is made of a woven material having a pore size in the range between about 15 and 40 micron.

16. The filter (1) according to claim 1, wherein the filtering element (4) has an outer diameter of less than about 50 mm and has a filtering surface of at least about 560 cm².

17. The filter (1) according to Claim 1, wherein the filtering element (4) includes alternating short pleats (b) and long pleats (a) configured such that a filter priming volume decreases significantly with respect to a configuration having pleats of a generally equal length.

18. Method for filtering blood comprising:
- providing a blood filter (1) according to any one of claims 1 to 17.
- flowing blood in a fluid line from the inlet port (2) to the outlet port (5), wherein blood traverses the filtering element (4);
whereby any gases contained in the blood are removed from blood via the vent port (6).

## Patentansprüche

1. Blutfilter (1) aufweisend:
ein Gehäuse (3), welches einen Einlassanschluss (2), einen Auslassanschluss (5), einen Lüftungsanschluss (6) und ein Filterelement (4), welches hierin angeordnet ist, aufweist, wobei
das Filterelement (4) aus einem porösen, plissierten Material besteht, welches für den Kontakt mit Blut geeignet ist, eine Filtration des Blutes ermöglicht und in einer zylindrischen Konfiguration angeordnet ist, sodass es eine Folge von Falten(a, b, c) definiert, aufweisend erste Falten (a), welche eine erste Länge (Iₐ) aufweisen, und zweite Falten (b), welche eine zweite Länge (I_{b}) aufweisen, wobei
der Einlassanschluss (2) und der Auslassanschluss (5) in einer Fluidverbindung mit entgegengesetzten Oberflächen des Filterelements (4) stehen, und wobei
der Einlassanschluss (2) tangential an einer äußeren Umfangsoberfläche des Gehäuses (3) angeordnet ist,
**dadurch gekennzeichnet, dass**
die ersten Falten (a) und die zweiten Falten (b) eine unterschiedliche Länge aufweisen, wobei die erste Länge (lₐ) größer ist als die zweite Länge (l_{b}).

2. Filter (1) gemäß Anspruch 1, weiterhin aufweisend dritte Falten (c), wobei die dritten Falten(c) eine Zwischenlänge (l_{c}) aufweisen, welche zwischen der ersten Länge (lₐ) und der zweiten Länge (l_{b}) liegt.

3. Filter (1) gemäß Anspruch 1, wobei die Abfolge der Falten eine Wechselfolge einer ersten Falte (a) aufweist, welche zwischen zwei zweiten Falten (b) eingefügt ist.

4. Filter (1) gemäß Anspruch 1, wobei die Abfolge der Falten eine Wechselfolge einer ersten Falte (a) aufweist, welche zwischen zwei Paaren von zweiten Falten(b) eingefügt ist.

5. Filter (1) gemäß Anspruch 2, wobei die erste Länge (lₐ) größer ist als die zweite Länge (l_{b}) und die Zwischenlänge (l_{c}) zwischen der ersten Länge (lₐ) und der zweiten Länge (l_{b}) liegt,und wobei die Abfolge des Weiteren eine Wechselfolge einer dritten Falte (c) aufweist, welche zwischen zwei zweiten Falten (b) eingefügt ist.

6. Filter (1) gemäß Anspruch 5, wobei die Wechselfolge des Weiteren zwei erste Falten (a) aufweist, welche benachbart zu den zwei zweiten Falten (b) eingefügt ist.

7. Filter (1) gemäß Anspruch 1, wobei jede Falte (a, b, c) entfernte Enden aufweist, welche im Wesentlichen eine gemeinsame, angenommene äußere zylindrische Oberfläche des Filterelements (4) definieren.

8. Filter (1) gemäß Anspruch 1, wobei die ersten Falten(a) nahe Enden aufweisen, welche im Wesentlichen eine gemeinsame, angenommene innere zylindrische Oberfläche des Filterelements (4) definieren, und die zweiten Falten (b) nahe Enden aufweisen, welche im Wesentlichen eine gemeinsame, angenommene innere zylindrische Oberflächedes Filterelements (4) definieren.

9. Filter (1) gemäß Anspruch 1, wobei die Falten (a, b, c) symmetrisch sind und zwei Arme von im Wesentlichen gleicher Länge aufweisen.

10. Filter (1) gemäß Anspruch 1, wobei das Material eine Schichtstruktur mit mindestens einer Filterschicht aufweist.

11. Filter (1) gemäß Anspruch 10, wobei das Material eine Drei-SchichtStruktur mit einer zentralen Filterschicht zwischen zwei äußeren Unterstützungsschichten aufweist.

12. Filter (1) gemäß Anspruch 1, wobei die erste Länge (lₐ) ungefähr 15 mm beträgt und die zweite Länge (l_{b}) ungefähr 10 mm beträgt.

13. Filter (1) gemäß Anspruch 1, wobei der Auslassanschluss (5) an einem Bodenelement (10) und konzentrisch in Bezug auf die zentrale longitudinale Achse angeordnet ist.

14. Filter (1) gemäß Anspruch 1, weiterhin aufweisend einen Deckel (11), welcher an einem oberen Teil des Filterelements (4) angeordnet ist.

15. Filter (1) gemäß Anspruch 10, wobei die Filterschicht aus einem gewebten Material hergestellt ist und eine Porengröße im Bereich von ungefähr 15 bis 40 Mikrometer aufweist.

16. Filter (1) gemäß Anspruch 1, wobei das Filterelement (4) einen äußeren Durchmesser von weniger als ungefähr 50 mm und eine Filteroberfläche von mindestens ungefähr 560 cm² aufweist.

17. Filter (1) gemäß Anspruch 1, wobei das Filterelement (4) abwechselnde kurze Falten (b) und lange Falten (a) aufweist, welche so ausgelegt sind, dass ein Filterfüllvolumen in Bezug auf eine Konfiguration mit Falten einer im Wesentlichen gleichen Länge signifikant sinkt.

18. Verfahren zum Filtern von Blut, aufweisend:
- Bereitstellen eines Blutfilters (1) gemäß einem der Ansprüche 1 bis 17;
- Fließen von Blut in einer Strömungslinievon dem Einlassanschluss (2) zu dem Auslassanschluss (5), wobei das Blut durch das Filterelement (4) hindurchtritt;
wobei Gase, welche im Blut enthalten sind, von dem Blut über den Lüftungsanschluss (6) entfernt wird.

## Revendications

1. Filtre à sang (1) comprenant un boîtier (3) ayant un orifice d'entrée (2), un orifice de sortie (5), un orifice formant évent (6) et un élément filtrant (4) disposé à l'intérieur de ce dernier, dans lequel l'élément filtrant (4) est réalisé dans un matériau plissé poreux qui convient pour le contact avec le sang tout en fournissant sa filtration, et est agencé selon une configuration cylindrique, de sorte qu'il définit une séquence de plis (a, b, c) comprenant des premiers plis (a) ayant une première longueur (lₐ) et des deuxièmes plis (b) ayant une deuxième longueur (l_{b}), dans lequel l'orifice d'entrée (2) et l'orifice de sortie (5) sont en communication de fluide avec les surfaces opposées de l'élément filtrant (4), et dans lequel l'orifice d'entrée (2) est disposé de manière tangentielle sur la surface circonférentielle externe du boîtier (3), **caractérisé en ce que** les premiers plis (a) et les deuxièmes plis (b) ont une longueur différente, dans lequel la première longueur (lₐ) est supérieure à la deuxième longueur (l_{b}).

2. Filtre (1) selon la revendication 1, comprenant en outre des troisièmes plis (c), les troisièmes plis (c) ayant une longueur intermédiaire (l_{c}) entre la première longueur (lₐ) et la deuxième longueur (l_{b}).

3. Filtre (1) selon la revendication 1, dans lequel la séquence de plis comprend une alternance de chaque premier pli (a) intercalé entre deux deuxièmes plis (b).

4. Filtre (1) selon la revendication 1, dans lequel la séquence comprend une alternance de chaque premier pli (a) intercalé entre deux paires de deuxièmes plis (b).

5. Filtre (1) selon la revendication 2, dans lequel la première longueur (lₐ) est supérieure à la deuxième longueur (l_{b}) et la longueur intermédiaire (l_{c}) est entre la première longueur (lₐ) et la deuxième longueur (l_{b}), et en outre dans lequel la séquence comprend une alternance de chaque troisième pli (c) intercalé entre deux deuxièmes plis (b).

6. Filtre (1) selon la revendication 5, dans lequel l'alternance comprend en outre deux premiers plis (a) disposés de manière adjacente aux deux deuxièmes plis (b).

7. Filtre (1) selon la revendication 1, dans lequel chacun des plis (a, b, c) a des extrémités distales définissant généralement une surface cylindrique externe théorique commune de l'élément filtrant (4).

8. Filtre (1) selon la revendication 1, dans lequel les premiers plis (a) ont des extrémités proximales définissant généralement une première surface cylindrique interne théorique et les deuxièmes plis (b) ont des extrémités proximales définissant généralement une deuxième surface cylindrique interne théorique de l'élément filtrant (4).

9. Filtre (1) selon la revendication 1, dans lequel les plis (a, b, c) sont symétriques, comprenant deux bras de longueurs généralement identiques.

10. Filtre (1) selon la revendication 1, dans lequel le matériau présente une structure en couche comprenant au moins une couche filtrante.

11. Filtre (1) selon la revendication 10, dans lequel le matériau présente une structure à trois couches comprenant une couche filtrante centrale intercalée entre deux couches de support externes.

12. Filtre (1) selon la revendication 1, dans lequel la première longueur (lₐ) est d'environ 15 mm et la deuxième longueur (l_{b}) est d'environ 10 mm.

13. Filtre (1) selon la revendication 1, dans lequel l'orifice de sortie (5) est disposé sur un élément inférieur (10) et est disposé de manière concentrique par rapport à l'axe longitudinal central.

14. Filtre (1) selon la revendication 1, comprenant en outre un capuchon (11) positionné sur une partie supérieure de l'élément filtrant (4).

15. Filtre selon la revendication 10, dans lequel ladite couche filtrante est réalisée dans un matériau tissé ayant une taille de pore comprise entre environ 15 et 40 microns.

16. Filtre (1) selon la revendication 1, dans lequel l'élément filtrant (4) a un diamètre externe inférieur à environ 50 mm et a une surface filtrante d'au moins environ 560 cm².

17. Filtre (1) selon la revendication 1, dans lequel l'élément filtrant (4) comprend des plis courts (b) et des plis longs (a) alternés, configurés de sorte qu'un volume primaire de filtre diminue considérablement par rapport à une configuration ayant des plis d'une longueur généralement identique.

18. Procédé destiné à filtrer du sang, comprenant les étapes consistant à :
prévoir un filtre à sang (1) selon l'une quelconque des revendications 1 à 17 ;
laisser s'écouler le sang dans une conduite de fluide, de l'orifice d'entrée (2) à l'orifice de sortie (5), dans lequel le sang traverse l'élément filtrant (4) ;
moyennant quoi les gaz contenus dans le sang sont retirés du sang via l'orifice formant évent (6).
